# FASCICULE DE BREVET EUROPEEN

(11) **EP 1 644 318 B1**
(45) Date de publication et mention de la délivrance du brevet: **07.05.2008**
(21) Numéro de dépôt: 04767243.1
(22) Date de dépôt: 03.06.2004
(51) Int. Cl.: C07C 231/02, C07C 235/02, C07C 235/12, C12P 13/02

(54) **PROCEDE DE SYNTHESE DE COMPOSES DE TYPE CERAMIDE**
VERFAHREN ZUR SYNTHESE VON VERBINDUNGEN VOM CERAMIDTYP
METHOD FOR SYNTHESIZING CERAMIDE-TYPE COMPOUNDS

(30) Priorité: 03.06.2003 FR 0306661
(43) Date de publication de la demande: 12.04.2006
(73) Titulaire: Société La Biochimie Appliquée SA (Solabia), 93500 Pantin (FR)
(72) Inventeur: LASSALLE, Laurent, F-27780 Garennes Sur Eure (FR); YVERGNAUX, Florent, F-28100 Dreux (FR)
(74) Mandataire: Burtin, Jean-François
(86) Numéro de dépôt international: PCT/FR2004/001375
(87) Numéro de publication internationale: WO 2004/108659

(56) Documents cités:
- EP-A- 0 968 998
- BIOTECHNOLOGY AND BIOENGINEERING, vol. 75, no. 6, 2001, pages 676-681, XP001180633
- MAUGARD T ET AL: "Enzymatic synthesis of glycamide surfactants by amidification reaction" TETRAHEDRON, ELSEVIER SCIENCE PUBLISHERS, AMSTERDAM, NL, vol. 53, no. 14, 7 avril 1997 (1997-04-07), pages 5185-5194, XP004105565 ISSN: 0040-4020

## Description

La présente invention se rapporte au domaine de la chimie des corps gras et plus particulièrement aux procédés de synthèse enzymatique de composés de type céramide.

La présente invention réside essentiellement dans la synthèse de composés de type céramide de formule générale (I) : 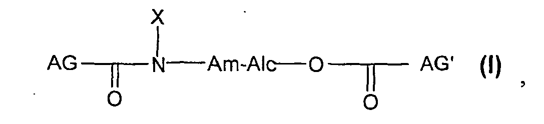 dans laquelle le groupement Am-Alc représente une chaîne hydrocarbonée préférentiellement saturée, linéaire, éventuellement ramifiée, comportant de 2 à 6 atomes de carbone, et issue d'un amino-alcool ; X représente un hydrogène ou une chaîne carbonée contenant de 1 à 4 atomes de carbone, éventuellement hydroxylée sur les carbones en position 2' et/ou suivants de la fonction amine ; et les groupements AG et AG' désignent chacun une chaîne hydrocarbonée, saturée ou insaturée, issue d'un acide gras et comportant de 4 à 30 atomes de carbone et éventuellement hydroxylé ; les deux groupements AG et AG' pouvant être identiques ou différents.
Plus particulièrement, la présente invention a pour objet un nouveau procédé de synthèse enzymatique de ces composés de type céramide, à partir d'acides gras et/ou d'esters d'acide gras, et d'amino-alcools, comportant au moins une étape d'amidification et une étape d'estérification, toutes deux réalisées par voie enzymatique, dans un ordre indifférent.

Les céramides figurent parmi les constituants lipidiques les plus importants du stratum corneum. Ils sont constitués d'une longue chaîne carbonée, liée à un acide gras par l'intermédiaire d'une liaison amide. Ils sont naturellement présents à l'état de traces au niveau des tissus où ils ont des effets biologiques importants.

Exerçant un rôle vital dans le maintien de la perméabilité des tissus en eau, les céramides sont étroitement liées au vieillissement cutané. En effet, on sait que l'apparence de la peau est essentiellement liée à la constitution en eau de ses différentes couches. Or l'altération des lipides membranaires, notamment du fait de l'utilisation de détergents souvent responsables de leur élimination, a pour conséquence d'augmenter la perte en eau. En outre, cette destruction de la barrière cutanée conduit à une augmentation de la sensibilité cutanée et à une irritation potentielle.- (Kersher M. et al., Eur. J. Dermatol., 1991, 139-43; Imokawa G..et al, J.Soc-Cosmet.Chem., 1989, 40, 273-285). De nombreuses études, telles que celles mentionnées dans les brevets US 5,476,671, WO95/34531 ou dans la publication R.D. Petersen, Cosm. Toil., 1992, 107, 45 ont montré que l'application topique de céramides permet de compenser leur élimination, ou leur dégradation. Ces composés sont donc d'une grande utilité, particulièrement dans le domaine de la cosmétologie et de la dermatologie.

Constituants naturels de presque tous les êtres vivants, les céramides peuvent être obtenus par extraction à partir d'animaux (Lambes H., 2nd ASCS, 1995, 106-125), de plantes (WO 9221321, Rousset G., Inocosm. Lab) ou de levures (WO 9410131, Casey J. et al., Unilever). Cependant, ces procédés d'extraction sont souvent longs et limités par la disponibilité des sources naturelles. Ceci augmente, en outre, le coût des céramides ainsi obtenus.

De nombreux analogues des céramides naturels, dénommés pseudo-céramides, ont de ce fait été synthétisés par voie chimique. Les pseudo-céramides ou composés de type céramide ressemblent aux céramides, sans toutefois leur être identiques. Ainsi, dans le brevet EP028281 (Ohashi Yukihiro et al., Kao Corp., 1988), on a décrit un procédé de synthèse de pseudo-céramides par réaction entre un éther de glycidyle et un amino-alcool, lequel est ensuite substitué par un acide gras via une liaison amide. Trois étapes consécutives sont ainsi requises pour l'obtention du pseudo-céramide.

Dans le brevet US 5,221,757 (Ohashi Yu kihiro et al., Kao Corp, 1993), on a également décrit' une synthèse similaire de pseudo-céramides, en 2 à 6 étapes, par réaction entre du glycidyl éther, un amino-alcool et un acide gras. Ce procédé de synthèse s'avère cependant très coûteux, relativement compliqué et ne constitue pas une solution satisfaisante pour la synthèse de structures variées.

Un autre brevet WO 92/03129 (Hannun Yusuf et al., Univ-Duke, 1992) expose la synthèse de pseudo-céramides ayant une structure proche des céramides naturels. Cependant, ce procédé est trop complexe pour pouvoir être exploité à l'échelle industrielle.

Dans la demande de brevet FR 2 757 853(Pacific Corporation, 1998), on a exposé un procédé de synthèse chimique de composés également très proches des céramides naturels. Ce document énumère en outre, sans plus de précision, des structures de céramides naturels dérivés de sphingosine et donc hydroxylés en position alpha sur les deux chaînes.

Selon une toute autre méthode décrite dans le brevet EP 0884 305 (L'Oréal, 1998), des dérivés de type céramide ont pu être synthétisés par réaction, sous irradiation par micro-ondes, d'un acide gras avec un amino-alcool de structure définie. Cependant, une telle synthèse s'effectue à une température élevée, ce qui n'est pas toujours adapté à certains composés fragiles, tels que les dérivés d'acides gras insaturés.

D'une manière générale, les procédés de synthèse chimique sont en outre peu sélectifs, comportent de multiples étapes, nécessitent bien souvent des réactions intermédiaires de protection de certaines fonctions et requièrent des réactifs coûteux et/ou toxiques. Ainsi, le brevet EP0 968 998 (Elf Atochem SA) décrit un procédé de synthèse chimique de composés analogues de céramides à partir d'agent acylant et d'amino-alcool. Ce procédé de synthèse présente cependant l'inconvénient d'être très peu sélectif, de sorte qu'un rendement molaire inférieur à 75% est obtenu. Il est mentionné que les étapes de synthèse peuvent être également effectuées par voie enzymatique et en particulier par des estérases classiques notamment les lipases et les protéases, sans plus de précision quant à leur mise en oeuvre ou quant à un avantage escompté pour cette voie.

La voie biotechnologique par synthèse enzymatique des composants de type céramide a été en effet largement explorée. Ainsi, on a décrit dans le brevet WO 942 6919 (Gist Brocades, 1997) un procédé de synthèse utilisant une lipase de *Pseudomonas alcaligenes* pour réaliser la liaison amide entre la phytosphingosine et le stéarate de méthyle. Cependant, cette réaction requiert un solvant bien particulier, de préférence du tetrahydrofurane (THF). De ce fait, une telle voie de synthèse est très coûteuse et donc peu appropriée à une utilisation industrielle. D'autres publications décrivent l'utilisation de lipases pour la production de liaisons amide dans un solvant organique, notamment celles de Zaks A. et al:(Proc. Natl. Acad. Sci. U.SA., 1985, 82, 3192-3196) et Margolin A. L. et al.(J. Am. Chem. Soc., 1987, 109, 3802-3804.) Toutefois, dans cette étude, la réaction d'amidation de lysosphingolipides, classe d'amino-alcools nécessaire à la synthèse de céramides, s'est révélée infructueuse, quelle que soit la nature de la lipase. L'utilisation de lipases sur des réactifs possédant plusieurs groupements fonctionnels tels que les amino-alcools a également été rapportée dans la littérature. Plusieurs facteurs pourraient ainsi influencer fortement la formation des produits, notamment le type de lipase, le type de substrat mais également le type de solvant utilisé.

Des études réalisées par Bistline R. G. et al.,(JAOCS, 1991, 68, 95-98) ainsi que par Djeghaba Z. et al, (Tetrahedron Lett., 1991, 32, 761-762) ont d'ailleurs démontré que la nature du solvant pouvait avoir une influence sur l'activité et là sélectivité de l'enzyme lors des réactions d'amidation. De même, Montet D. et al. (Revue Française dés Corps Gras, 1989, 36, 79-83) a montré que, lors de la réaction d'acylation de l'amino-propanol par la lipase de *Mucor miehei* dans un solvant organique, la sélectivité est fortement influencée par la nature du solvant. Il faut noter que l'utilisation de cette lipase n'a pas permis d'effectuer la réaction d'amidation avec le lysosphingolipide. Toutes les lipases ne sont effectivement pas capables de réaliser la liaison amide nécessaire à la synthèse de céramides. Ceci a notamment été montré dans une autre publication de Montet D. et al. (Fat Sci. Technol., 1989, 91, 14-18). Un document brevet (EP 0298796, Graille, J. et al.) mentionne également ce résultat et décrit un procédé permettant d'effectuer une amidation notamment avec l'enzyme de *Mucor miehei.* Malgré des conditions très définies, les rendements obtenus avec cette conversion restent très variables, comme en témoignent les tests effectués, et avec un maximum d'environ 80% de conversion. De tels rendements sont insuffisants pour permettre une exploitation rentable de ces réactions enzymatiques.

Ainsi, aucun des procédés décrits dans l'art antérieur n'avait jusqu'alors permis de synthétiser des composés de type céramide, de manière satisfaisante. Or, de tels composés présentent un grand intérêt également pour l'industrie des amphiphiles en général, dans des domaines aussi divers que les produits tensioactifs, des agents mouillants, des produits anticorrosion etc...., ces molécules pouvant trouver des applications aussi bien industrielles, ménagères, cosmétiques que pharmaceutiques. Il existait donc un besoin réel et permanent de trouver un procédé de synthèse de composés de type céramide qui soit à la fois simple, efficace, économique et compatible avec des conditions industrielles.

Ce problème technique a été résolu, de manière particulièrement surprenante, selon la présente invention, par un procédé de synthèse comprenant au moins une étape d'amidification effectuée par l'enzyme Lipase B de *Candida antartica* et une étape d'estérification également réalisée par une enzyme de type lipase.

A la différence de l'art antérieur, un tel procédé est remarquable en ce qu'il comporte seulement deux étapes, simples et rapides, ne nécessite ni solvant, ni purification et s'effectue avec un rendement quantitatif pour chacune de ces deux étapes. L'utilisation d'enzymes permet, en outre, d'obtenir une très grande sélectivité dans le produit désiré. Ce procédé constitue donc une solution aux difficultés rencontrées jusqu'à maintenant pour la synthèse des composés de type céramide.

La présente invention a pour objet un procédé de synthèse de composés de type céramide comportant au moins une étape d'amidification effectuée par l'enzyme Lipase B de *Candida antartica* et une étape d'estérification, également réalisée par une enzyme de type lipase, lesdits composés de type céramide étant de formule générale (I) : 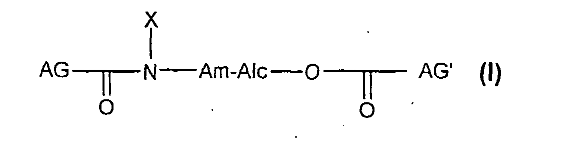 dans laquelle le groupement Am-Alc représente une chaîne hydrocarbonée saturée, linéaire, éventuellement ramifiée, comportant de 2 à 6 atomes de carbone, issue d'un amino-alcool ; X représente un hydrogène ou une chaîne carbonée contenant de 1 à 4 atomes de carbone, éventuellement hydroxylée sur les carbones en position 2' et/ou suivants de la fonction amine ; et les groupements notés AG et AG' désignent chacun une chaîne carbonée, saturée ou insaturée, éventuellement hydroxylée et comportant de 4 à 30 atomes de carbone, issue d'un acide gras et/ou d'un ester d'acide gras ; les deux groupements AG et AG' pouvant être identiques ou différents.

Les composés de type céramide obtenus selon la présente invention, constituent une catégorie particulière de pseudo-céramides. Par pseudo-céramides, on entend généralement des composés comportant une fonction amide et une autre liaison. Selon la présente invention, les pseudo-céramides de formule (I) se distinguent par le fait que la liaison supplémentaire est de type ester. Cette liaison ester est spécifiquement orientée dans la molécule. Ainsi, ces composés de type céramide sont particulièrement proches des céramides naturels ou des pseudo-céramides issus de synthèses chimiques. Du fait de leurs groupements chimiques similaires, ils présentent ainsi souvent les mêmes propriétés.

Par rendement quantitatif, on entend selon l'invention un rendement supérieur à 93%. Un rendement quasiment de 100% est même obtenu lors de l'étape d'amidification.

Les deux étapes, ci-après décrites plus amplement, sont indépendantes et peuvent être effectuées successivement et/ou simultanément, dans un ordre différent, sans que cela n'ait de conséquence sur la structure du produit synthétisé.

Selon l'invention, on entend par étape d'amidification, l'étape qui consiste à faire réagir un amino-alcool, avec un acide gras et/ou un ester d'acide gras. Lorsqu'il s'agit de la première étape, elle peut être représentée par le schéma réactionnel (A) suivant, menant à obtention de composés intermédiaires de formule (II):

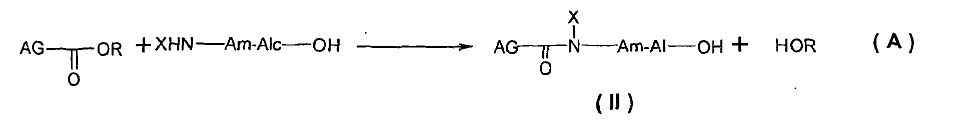

Selon l'invention, R désigne un hydrogène ou une chaîne carbonée contenant de 1 à 5 atomes de carbone, tels que notamment un groupement méthyle, éthyle, propyle, butyle, pentyle, éventuellement substitué tel qu'un groupement glycérol (trihydroxypropane). Cependant, R peut être aussi tout groupement chimique, pour autant qu'il ne provoque pas d'encombrement stérique susceptible de nuire à l'accessibilité de la fonction carboxyle de l'acide gras ou ester de l'estér d'acide gras. En outre, R sera choisi de préférence de telle sorte que l'alcool ROH formé soit volatile, comme par exemple l'éthanol, l'isopropanol. Dans le cas contraire, une étape de purification sera nécessaire et pourra par exemple se faire par chromatographie liquide à haute performance (HPLC), par chromatographie sur colonne de silice ou par cristallisation.

Lors de cette étape d'amidification, la liaison amide est formée entre la fonction carbonyle d'un acide gras ou l'ester correspondant, et la fonction amine primaire ou secondaire d'un amino-alcool ou d'un ester d'amino-alcool, par une enzyme de type lipase. Plus particulièrement, on utilise la lipase B de *Candida antartica,* appartenant aux classes des triacylglycérol hydrolases et carboxylestérases (classe enzymatique EC 3.1.1.3). Il peut notamment s'agir de la lipase commercialisée par la société Novozymes SA, sous la dénomination Novozym® 435, produite par fermentation du microorganisme génétiquement modifié *Aspergillus oryzae* et avantageusement immobilisée sur support. La mise en oeuvre n'est pas limitée à l'usage de l'enzyme commercialisée présentement par cette Société. Cependant, il convient de noter qu'il doit s'agir de ce type de lipase pour obtenir un bon rendement, conformément à la présente invention.

De manière préférentielle, lors de l'amidification, on utilisera les réactifs en conditions stoechiométriques de façon à ce que, dans ces proportions, l'intégralité des réactifs soit consommée, afin d'éviter les réactifs résiduels.

Par étape d'estérification, on entend selon l'invention, la réaction par laquelle un acide gras ou un ester d'acide gras est greffé sur la fonction alcool libre, terminale ou non, d'un amino-alcool. Considérant le cas où cette étape fait suite à l'amidification, elle peut être représentée par le schéma réactionnel (E) suivant: 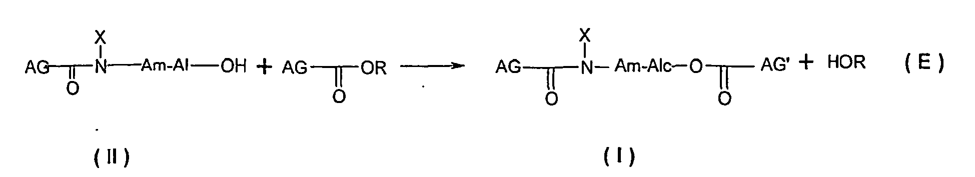 Selon l'invention, R désigne un hydrogène ou une chaîne carbonée comportant de 1 à 5 atomes de carbone, tels que notamment un groupement méthyle, éthyle, propyle, butyle, isobutyle, terbutyle, pentyle, isopentyle, éventuellement substitué tel qu'un groupement glycérol (trihydroxypropane) ou triméthylolpropane. Cependant, R pourrait être tout groupement chimique inerte pour autant qu'il ne provoque pas d'encombrement stérique susceptible de nuire à l'accessibilité de la fonction carboxyle de l'acide gras ou ester de l'ester d'acide gras. En outre, comme pour l'amidification, R doit être tel que l'alcool ROH formé soit volatil tel que l'éthanol, l'isopropanol. Dans le cas contraire, une étape de purification serait nécessaire et pourrait se faire, par exemple, par distillation.

Lors de cette étape d'estérification, la liaison ester est réalisée entre le groupement hydroxyle du composé de formule (II) (amide-alcool), et le groupement carboxyle de l'acide gras ou l'ester d'acide gras, saturé ou insaturé, par un enzyme de type lipase et plus particulièrement par une lipase appartenant à la classe des triacylglycérol hydrolases (classe enzymatique EC 3.1.1.3).

Lors de cette étape, on pourra, de façon plus générale, utiliser toute enzyme capable de catalyser spécifiquement ces réactions de liaisons ester. Cependant, on préfère en particulier la lipase de *Rhizomucor miehei.* En effet, il a également été découvert que cette enzyme ne requiert pas de solvant et du fait de sa spécificité, présente l'avantage d'effectuer la conversion en ester avec un très bon rendement. Elle participe ainsi à l'amélioration du rendement du procédé global de synthèse des céramides selon la présente invention. En particulier, mais sans que cela soit limitatif, on peut utiliser la lipase commercialisée par la société Novozymes SA sous la dénomination Lipozyme® RM IM, également produite par fermentation du microorganisme génétiquement modifié *Aspergillus oryzae* et avantageusement immobilisée sur support.

Selon un mode particulièrement intéressant de mise en oeuvre du procédé, on utilise la lipase B de *Candida antartica* lors de l'étape d'amidification et la lipase de *Rhizomucor miehei* lors de l'étape d'estérification. Ces deux enzymes possèdent, en effet, une très forte sélectivité. Ainsi, Novozym® 435 ne réalise que l'étape d'amidification et Lipozyme® RM IM que l'étape d'estérification. Il est important alors de noter que l'enchaînement des étapes avec ces deux enzymes peut être inversé sans affecter la nature du produit et avec un rendement similaire.

En outre, on pourra même effectuer ces deux étapes de façon simultanée. Si on utilise un seul type d'acide gras, les chaînes AG et AG' étant identiques, un seul composé de type céramide de formule I sera obtenu, comportant deux groupements AG et AG' identiques. En revanche, en utilisant deux types ou plus d'acides gras, on obtiendra un mélange de composés de type céramide, tous de formule (I) mais contenant les différentes combinaisons possibles des différents groupements carbonés AG et AG'.

Lors de chacune de ces réactions, on utilise avantageusement des lipases immobilisées sur un support organique inerte, ce qui permet de les éliminer aisément du milieu réactionnel et de pouvoir ensuite les recycler. De manière préférentielle, elles seront adsorbées sur une résine macroporeuse telles que le sont les enzymes Novozym® 435 et Lipozyme®.RM IM. Le coût de ce procédé est, en effet, très avantageusement réduit par recyclage de l'enzyme. Il a ainsi été constaté que Novozym® 435 peut être recyclée environ dix fois sans perte d'activité.pour la réaction d'amidification selon ce procédé.

Plus précisément, le procédé de synthèse selon l'invention peut être mis en oeuvre de la manière préférentielle suivante :
- l'étape de formation de l'amide a lieu en mélangeant, dans des conditions stoechiométriques, l'acide gras ou l'ester correspondant, avec un amino-alcool, en présence d'une lipase telle que Novozym® 435. La réaction est réalisée à une température comprise entre 40 et 100°C, et préférentiellement entre 55 et 85°C. On peut l'effectuer à la pression atmosphérique ou en utilisant un vide compris entre 500 et 1 mbars, préférentiellement compris entre 200 et 30 mbars. Pour obtenir un rendement quantitatif minimal d'au moins 93% environ dans ces conditions, la réaction est poursuivie pendant au moins 16 heures, et de préférence pendant au moins 20 heures.
- l'étape de formation de la fonction ester est effectuée en faisant réagir l'amide obtenu lors de la première étape avec un acide gras ou l'ester correspondant, en présence d'une lipase telle que Lipozyme® RM IM. Le ratio ester d'acide gras sur l'amide-alcool est compris entre 1 et 2, mais préférentiellement entre 1 et 1,5. La réaction est réalisée à une température comprise entre 40 et 90°C, de préférence entre 50 et 70°C. Elle est effectuée à pression atmosphérique
ou en utilisant un vide partiel compris entre 500 et 1 mbars et préférentiellement entre 200 et 30 mbars. Pour obtenir un rendement quantitatif minimal de l'ordre de 93% dans ces conditions, la réaction est poursuivie pendant au moins 18heures, et de préférence pendant au moins 24 heures.

De façon avantageuse, on réalise chaque étape sous un vide partiel compris entre 200 et 30mbars. En effet, il s'avère que l'utilisation du vide permet d'éliminer l'eau ou l'alcool formé lors de la condensation au fur et à mesure, et d'accélérer considérablement la cinétique de la réaction. En outre, par le vide, on limite la dégradation d'un grand nombre d'acides gras oxydables.

Pour les raisons précédemment expliquées, on doit insister, en outre, sur le fait que l'ordre d'enchaînement des deux réactions avec ces deux enzymes n'est donné qu'à titre d'exemple et ne doit pas être considéré comme limitatif Il est laissé à l'initiative de l'expérimentateur.

Ainsi, selon une variante de réalisation, la synthèse peut être effectuée selon une première étape d'estérification qui peut être représentée par le schéma réactionnel (E') suivant:

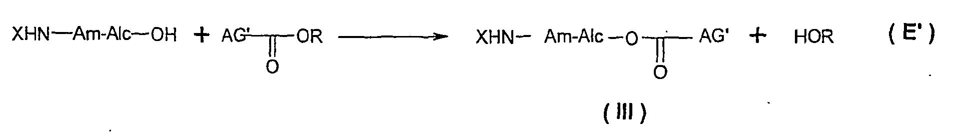

Cette étape d'estérification s'effectue entre un amino-alcool, et un acide gras et/ou ester d'acide gras afin d'obtenir un amino-ester d'acide gras, dans les conditions précédemment décrites pour effectuer la réaction E, favorables à l'estérification.

La deuxième étape sera la réaction d'amidification qui peut être représentée par le schéma réactionnel (A') suivant: 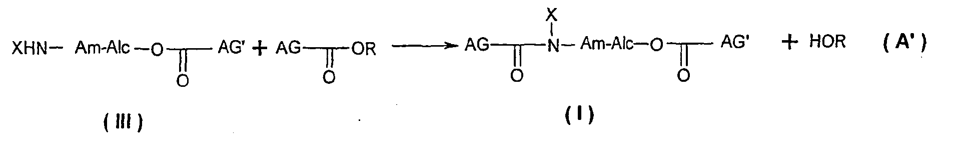 Cette deuxième étape de synthèse est réalisée entre l'amino-ester d'acide gras précédemment obtenu et un acide gras ou l'ester d'acide gras, dans les conditions précédemment décrites pour effectuer la réaction A.

Lorsqu'on utilise la lipase B de *Candida antartica* et la lipase de *Rhizomucor miehei,* aucun solvant n'est nécessaire durant chacune des étapes de synthèse des pseudo-céramides. En effet, à une température supérieure à 65°C environ lors de chaque réaction, les réactifs et les produits sont sous forme liquide, ce qui leur permet d'exercer également une fonction de solvant. Ceci permet avantageusement de se dispenser d'une étape de purification finale des composés (I) ainsi formés.

Il convient de noter qu'on obtient les composés de formule (I) sous forme de mélange avec des acides gras et/ou des esters d'acide gras résiduels comme par exemple le palmitate d'éthyle. Ce composé résiduel ne constitue pas un inconvénient pour une formulation sous forme de compositions cosmétiques ou dermatologiques. Néanmoins, si nécessaire, le composé de formule (I) peut être purifié selon les méthodes habituelles de séparation telles que notamment par chromatographie liquide à haute performance (HPLC)ou par passage sur colonne de silice ou de cellulose, ou par cristallisation.

Le procédé selon l'invention comprend ainsi avantageusement un nombre réduit d'étapes, au minimum une ou deux (amidification et estérification, de manière simultanée ou successive). Les-composés de formule (I) étant particulièrement stables, d'autres étapes peuvent cependant être ajoutées, notamment après les deux étapes d'amidification et d'estérification, telles que la formation de dérivés d'alcoylation, d'oxydation ou de réduction.

Dans le cas où on souhaite utiliser un solvant notamment l'éther tertiobutylique (TBEE) ou l'hexane, la synthèse s'effectuera sous pression atmosphérique.

Les températures maximales des réactions sont déterminées par les capacités de recyclage des enzymes. De manière générale, l'homme du métier comprendra que les conditions de synthèse peuvent varier en fonction de plusieurs facteurs, notamment de la concentration en enzymes et en réactifs, du pH, de la température. Lors de chaque étape, on essaiera de se rapprocher des conditions optimales d'activité des enzymes. A titre d'exemple, mais sans que cela ne soit limitatif, on se référera aux procédés décrits dans les exemples 1 à 21.

La poursuite de chaque réaction au delà du temps indiqué pour les conditions précitées ne présente pas d'inconvénient mais elle ne conduit pas nécessairement à une augmentation du rendement.

Les acides gras et /ou esters d'acides gras utilisables selon le procédé de la présente invention ont une chaîne carbonée AG ou AG', préférentiellement linéaire, contenant entre 4 et 30 atomes de carbone, saturée ou insaturée, et éventuellement hydroxylée. De préférence, on choisira ceux qui sont constitués de 10 à 22 atomes de carbone. En outre, de préférence, les acides gras ne portent pas d'hydroxylation en alpha de la fonction carboxylique ou ester de l'acide gras, afin d'éviter les acylations parallèles, notamment lors de l'estérification.

A titre d'exemples d'acides gras préférés, on peut notamment citer :
- l'acide caprique (acide décanoïque)
- l'acide undécanoïque
- l'acide laurique (acide dodécanoïque)
- l'acide myristique (acide tétradécanoïque)
- l'acide palmitique (acide hexadécanoïque)
- l'acide stéarique (acide octadécanoïque)
- l'acide oléique (acide octadéca-9-énoïque)
- l'acide ricinoléique (acide 12-hydroxy-octadéca-9-énoïque)
- l'acide linoléique (acide octadéca-9,12-diénoïque)
- l'acide linolénique (α : acideoctadeca-9,12,15-triénoïque ou γ: acide octadeca-6,9,12-triénoïque)
- l'acide eicosapentaènoïque (acide eicosa-5,8,11,14,17-pentaénoïque)
- l'acide docosahexaènoïque (acide docosa-4,7,10,13,16,19-hexaénoïque)
- l'acide stéaridonique (acide octadéca-6,9,12,15-tétraénoïque)
- l'acide aleuritolique (acide 9,10,16-trihydroxy hexadécanoïque)

Parmi les esters d'acides gras utilisables, on peut notamment citer les esters méthylique, éthylique, propylique, isopropylique. terbutylique,...Les dérivés phosphorylés tels que certains dérivés de sphingosine sont en revanche exclus.

Les acides gras ou les esters d'acides gras peuvent être utilisés purs ou sous forme d'un mélange d'acides gras, notamment extraits de micro-algues tel que la spiruline, d'une huile végétale, telle que par exemple, l'huile d'onagre, de bourrache, ou d'huile animale telles que les huiles de poissons. Il pourra notamment s'agir d'un mélange d'acides gras provenant de la saponification d'huiles.

Les amino-alcools utilisables, selon le procédé de la présente invention, possèdent un ou plusieurs groupements hydroxyles primaires ou secondaires. La fonction amine peut être primaire ou secondaire. Pour optimiser le rendement, on utilisera préférentiellement un amino-alcool possédant une fonction amine primaire.

Les amino-alcools utilisables, selon le procédé de la présente invention, répondent à la formule générale (IV) : 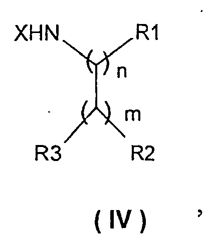 dans laquelle :
- n est un nombre entier choisi parmi les valeurs 1, 2 et 3, et m est un nombre entier choisi parmi les valeurs 1, 2 et 3.
- X est choisi parmi l'hydrogène et une chaîne carbonée contenant de 1 à 4 atomes de carbone, éventuellement hydroxylée sur les carbones en position 2' et/ou suivants de la fonction amine, -R1 est choisi parmi l'hydrogène, et une chaîne alkyle comportant de 1 à 4 atomes de carbone, de préférence saturée, linéaire et éventuellement ramifiée et/ou hydroxylée,
- R2 est choisi parmi l'hydrogène, un hydroxyle, un groupement NH₂ et une chaîne alkyle comportant de 1 à 4 atomes de carbone, de préférence saturée, linéaire, éventuellement ramifiée et/ou hydroxylée,
- R3 est choisi parmi l'hydrogène, un hydroxyle et un groupement CH₂OH et dans laquelle, au moins l'un des groupements R1, R2 ou R3 contient un groupement hydroxyle. Ce procédé, par les enzymes très sélectives qu'il met en oeuvre, présente l'avantage de permettre la synthèse de composés de type céramide de formule (I) avec un très bon rendement, y compris lorsque le réactif amino-alcool ne contient qu'un seul groupement hydroxyle. Ainsi selon un mode préférentiel, un seul groupement R1, R2 ou R3 contient un groupement hydroxyle.

Les amino-alcools sont donc linéaires, éventuellement ramifiés, de préférence saturés et contiennent de 2 à 6 atomes de carbone. De préférence, ils sont partiellement solubles dans l'acide gras ou dans son ester d'acide gras qui constitue le réactif initial. A titre d'exemples d'amino-alcools préférés, on peut notamment citer :
- 1-amino-2-propanol
- 2-amino-1-propanol
- 3-amino-1-propanol
- 2-(methylamino)ethanol
- 1-amino-2-butanol
- 2-amino-1-butanol
- 3-amino-1-butanol
- 4-amino-1-butanol
- 2-amino-2-methyl-1-propanol
- 2-(ethylamino)ethanol
- 2-amino-3-methyl-1-butanol
- 1-amino-2-pentanol
- 2-amino-1-pentanol
- 5-amino-1-pentanol
- 2-(propylamino)ethanol
- 1-amino-2-hexanol
- 2-amino-1-hexanol
- 6-amino-1-hexanol
- diethanolamine
- 3-amino-1,2-propanediol
- 2-amino-1,3-propanediol
- 2-amino-2-methyl-1,3-propanediol
- 2-amino-2-ethyl-1,3-propanediol
- 2-amino-2-hydroxymethyl-1,3-propanediol
- N-2,3,4,5,6-pentahydroxyhexylamine

Les composés de type céramide obtenus directement par ledit procédé de synthèse selon l'invention possèdent la structure générale (I) : 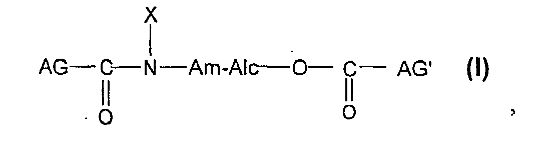 dans laquelle Am-Alc désigne une chaîne carbonée préférentiellement saturée, linéaire, éventuellement ramifiée, comportant de 2 à 6 atomes de carbone, préférentiellement non hydroxylée ; X représente un hydrogène ou une chaîne carbonée contenant de 1 à 4 atomes de carbone, éventuellement hydroxylée sur les carbones en position 2' et/ou suivants de la fonction amine; les groupements AG et AG' représentant chacun une chaîne carbonée, saturée ou insaturée, éventuellement hydroxylée, comportant de 4 à 30 atomes de carbone, préférentiellement de 10 à 22 carbones, les deux groupements AG et AG' pouvant être identiques ou différents.

Ces composés de formule (I) contiennent donc nécessairement deux groupements oxy, séparés par au moins une liaison amide et une liaison ester, et par une chaîne carbonée Am-Alc. Ils contiennent en outre deux chaînes carbonées AG et AG' pouvant être identiques ou différentes selon les acides gras et/ou esters d'acides gras utilisés comme réactifs.

Les composés de formule (I) ont un point de fusion de l'ordre de 60 à 65°C ce qui permet de les synthétiser à température élevée et de faciliter leur incorporation dans les formulations. Ces composés ont par ailleurs de bonnes propriétés de conservation, supérieures à celles de l'amide simple correspondant de formule (II).

Le procédé peut être utilisé sur des composés optiquement actifs. Cette sélectivité est en effet conservée dans le composé final (I). Lorsqu'ils présentent au moins un atome de carbone asymétrique, ces nouveaux composés peuvent être dédoublés en leurs isomères ou leurs diastéréoisomères erythro et threo.

Parmi les nouveaux composés de type céramide objet de l'invention, on peut notamment citer, non limitativement, à titre de composés préférés :
- (3-decanoyloxy)-2-hydroxy propyl amide de l'acide octadéca-9,12-cis-cis-diènoïque
- (3-dodecanoyloxy)-2-hydroxy propyl amide de l'acide octadéca-9,12-cis-cis-diènoïque
- (3-tétradécanoyloxy)-2-hydroxy propyl amide d'acide octadéca-9,12-cis-cis-diènoïque
- (3-hexadécanoyloxy)-2-hydroxy propyl amide d'acide octadéca-9,12-cis-cis-diènoïque
- (3-octadécanoyloxy)-2-hydroxy propyl amide d'acide octadéca-9,12-cis-cis-diènoïque
- (3-octadéca-9-diénoyloxy)-2-hydroxy propyl amide d'acide octadéca-9,12-cis-cis-diènoïque
- (3-octadéca-9,12-cis-cis-diènoyloxy)-2-hydroxy propyl amide de l'acide octadéca-9,12-cis-cis-diènoïque
- (3-octadéca-12-hydroxy-9 diènoyloxy)-2-hydroxy propyl amide de l'acide octadéca-9,12-cis-cis-diènoïque
- (3-eicosa-5,8,11,14,17-pentaènoyloxy)-2-hydroxy propyl amide de l'acide octadéca-9,12-cis-cis-diènoïque
- (3-docosa-4,7,10,16,19-hexaènoyloxy)-2-hydroxy propyl amide de l'acide octadéca-9,12-cis-cis-diènoïque

Lorsque l'étape d'amidification selon ledit procédé est la première étape de synthèse, les composés intermédiaires amides sont obtenus et ont la formule générale (II) : 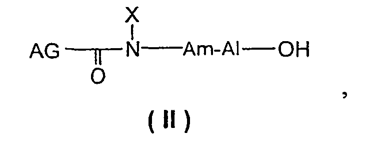 dans laquelle le groupement Am-Alc représente une chaîne hydrocarbonée saturée, linéaire, éventuellement ramifiée, comportant de 2 à 6 atomes de carbone, dérivée d'un amino-alcool ; X représente un hydrogène ou une chaîne carbonée contenant de 1 à 4 atomes de carbone, éventuellement hydroxylée sur les carbones en position 2' et/ou suivants de la fonction amine; et le groupement AG désigne une chaîne hydrocarbonée linéaire, saturée ou non, comportant de 4 à 30 atomes de carbone, préférentiellement de 10 à 22 atomes de carbones et issue d'un acide gras.

Ces composés intermédiaires sont obtenus par amidification entre la fonction carboxyle des acides gras et/ou des esters d'acides gras et la fonction amine des amino-alcools selon le schéma réactionnel (A) du procédé objet de la présente invention. Cette étape est ainsi réalisée par la lipase B de *Candida antartica* dans les conditions précédemment décrites.

Ces composés intermédiaires sont particulièrement intéressants en ce qu'ils permettent d'obtenir les nouveaux composés de type céramide en une seule étape d'estérification (E) décrite selon la présente invention.

De tels composés intermédiaires amides se dégradent cependant rapidement. C'est pourquoi il est souhaitable d'effectuer rapidement la réaction d'estérification conduisant aux pseudo-céramides (I) qui sont des composés plus stables.

Parmi ces nouveaux composés intermédiaires de formule (II), on peut notamment citer à titre d'exemples :
- (2-hydroxy-propyl) amide de l'acide octadéca-9,12-cis-cis-diènoïque
- (1-hydroxyméthyl-pxopyl) amide de l'acide octadéca-9,12-cis-cis-diènoïque
- (2,3-dihydroxy-propyl) amide de l'acide octadéca-9,12-cis-cis-diènoïque
- (2,3-dihydroxy-propyl) amide de l'acide décanoïque
- (2,3-dihydroxy-propyl) amide de l'acide dodécanoïque
- (2,3-dihydroxy-propyl) amide de l'acide tétradécanoïque
- (2,3-dihydroxy-propyl) amide de l'acide hexadécanoïque
- (2,3-dihydroxy-propyl) amide de l'acide octadécanoïque
- (2,3-dihydroxy-propyl) amide de l'acide octadéc-9-cis-ènoïque
- (2,3-dihydroxy-propyl) amide de l'acide octadéca-9,12-cis-cis-diènoïque
- (2,3-dihydroxy-propyl) amide de l'acide 12-hydroxy-octadéc-9-ènoïque
Les exemples 1 à 11 décrivent plus précisément les conditions de la réaction d'amidification.

Lorsque l'étape d'estérification selon ledit procédé est la première étape de synthèse, les composés intermédiaires esters sont obtenus et ont la formule générale (III) : 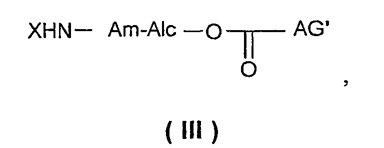 dans laquelle le groupement Am-Alc représente une chaîne hydrocarbonée saturée, linéaire, éventuellement ramifiée, comportant de 2 à 6 atomes de carbone, dérivée d'un amino-alcool ; X représente un hydrogène ou une chaîne carbonée contenant de 1 à 4 atomes de carbone, éventuellement hydroxylée sur les carbones en position 2'et/ou suivants de la fonction amine ; et le groupement AG' désigne une chaîne hydrocarbonée linéaire, saturée ou non, comportant de 4 à 30 atomes de carbone, préférentiellement de 10 à 22 atomes de carbone et issue d'un acide gras.

Ces composés intermédiaires sont obtenus par estérification entre la fonction carboxyle des acides gras et/ou des esters d'acides gras et la fonction hydroxyle des amino-alcools selon le schéma réactionnel (E') du procédé objet de la présente invention. Cette étape est ainsi réalisée par la lipase de *Rhizomucor miehei* dans les conditions précédemment décrites.

Ces composés intermédiaires sont particulièrement intéressants en ce qu'ils permettent d'obtenir les nouveaux composés de type céramides en une seule étape d'amidification (A') décrite selon la présente invention.

Parmi ces nouveaux composés intermédiaires de formule (III), on peut notamment citer à titre d'exemples :
- 1-amino-2-hydroxy propyl ester de l'acide décanoïque
- 1-amino-2-hydroxy propyl ester de l'acide dodécanoïque
- 1-amino-2-hydroxy propyl ester de l'acide tétradécanoïque
- 1-amino-2-hydroxy propyl ester de l'acide hexadécanoïque
- 1-amino-2-hydroxy propyl ester de l'acide octadécanoïque
- 1-amino-2-hydroxy propyl ester de l'acide octadéca-9-énoïque
- 1-amino-2-hydroxy propyl ester de l'acide octadéca-9,12-cis-cis-diènoïque
- 1-amino-2-hydroxy propyl ester de l'acide 12-hydroxy octadéca-9-énoïque
- 1-amino-2-hydroxy propyl ester de l'acide éicosa-5,8,11,14,17-pentaénoïque
- 1-amino-2-hydroxy propyl ester de l'acide docosa-4,7,10,16,19-pentaénoïque

Les composés de type céramide de formulé (I) peuvent être utilisés comme des composés actifs dans des compositions cosmétiques et/ou pharmaceutiques, et plus précisément dermatologiques. Ces compositions peuvent contenir de 0.01 à 90% desdits composés, de préférence de 0.1 à 30% en poids total de la composition et préférentiellement de 0.1 à 5%. De telles compositions peuvent contenir les véhicules usuels et cosmétiquement acceptables notamment comme agent diluant, agent dispersant, agent gélifiant, agent support de céramides, émollient solide, des gommes, des résines, des agents tensioactifs, des agents de protection solaire, des solvants, des charges telles que l'amidon de riz, des pigments, des conservateurs, des huiles essentielles, des agents antioxydants, des colorants, des pigments, des nacres, des parfums, des absorbeurs d'odeur, des agents régulateurs du pH ou des agents neutralisants, des épaississants, tels que ceux habituellement utilisés.

Les compositions se présentent sous une forme appropriée à l'application sur la peau et/ou les phanères telle que gel, lotion, notamment lotion capillaire et vernis, émulsion ou dispersion, notamment de type huile-dans-eau ou eau-dans-huile, crème notamment crème de mascara, onguent, lait, mousse, bâtons (stick), notamment sous forme coulée de baume à lèvres, rouge à lèvres.

Les composés obtenus par le procédé selon l'invention peuvent être formulés de manière similaire à ceux décrits dans l'art antérieur. Sans que cela ne soit limitatif, on pourra se reporter aux exemples de formulations fournies.

Sous forme d'émulsion, la composition selon l'invention contient des émulsionnants et des coémulsionnants habituellement utilisés par l'homme du métier. Des exemples d'émulsionnants et coémulsionnants appropriés sont des esters d'acides gras et de polyol tels que le stéarate de glycéryle, des esters d'acides gras et de polyéthylèneglycol tel que le stéarate de PEG-20. De manière avantageuse, la concentration en émulsionnant et en coémulsionnant est comprise entre 0.3 et 30% par rapport au poids total de la composition, de préférence, entre 0.5 et 5%.

Ces compositions sont ainsi utiles pour le soin et/ou le traitement et/ou la protection de l'épiderme et/ou de ses formations (cheveux, poils, ongles...) chez l'homme et/ou l'animal.En effet, en restaurant l'équilibre lipidique cutané, elles réparent les altérations de la couche protectrice cutanée et contrôlent les pertes en eau. Elles sont donc particulièrement utiles pour prévenir ou résoudre les problèmes de peau tels que la sécheresse, les rides et plis cutanées, les desquamations, les crevasses et gerçures et pour maintenir une peau souple, douce, hydratée et élastique et lutter contre les signes du vieillissement cutané. Les compositions cosmétiques ou dermatologiques ne présentent aucune toxicité et ne provoquent aucune intolérance locale. Elles ne sont pas non plus allergisantes.

Les exemples suivants sont présentés pour illustrer l'invention et ne doivent en aucun cas être considérés comme une limite à la portée de l'invention. Sauf mention contraire, les concentrations sont données en pourcentage par rapport au poids total de composition.

Dans les exemples ci-après, la réaction d'amidation est effectuée par la lipase B de *Candida antartica,* immobilisée sur support inerte utilisée sous la forme du produit commercialisé par la Société Novozyme SA sous la dénomination Novozym^{®} 435. Ce produit est thermostable, et présente une activité optimale à 40-80°C. Il a par ailleurs une activité d'estérification déclarée d'environ 10 000 unités de Propyl Laurate par gramme (PLU/g)

La réaction d'estérification est effectuée par la lipase de *Rhizomucor miehei,* immobilisée sur support inerte, utilisée sous la forme du produit commercialisé par la Société Novozym sous la dénomination Lipozyme^{®} RM IM. Ce produit présente une activité optimale de 30 à 70°C et a une activité d'environ 150 IUN/g.

### I. Exemple de réaction d'amidification à partir de différents amino-alcools (1-amino 2-propanol, 2-amino 1-butanol et 3-amino 1,2-propane diol) et en présence d'acide gras notamment l'acide linoléique.

| | |
|---|---|
| Exemple 1 | 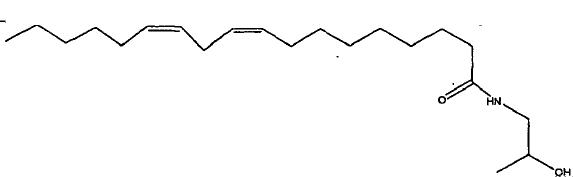 |
| Exemple 2 | 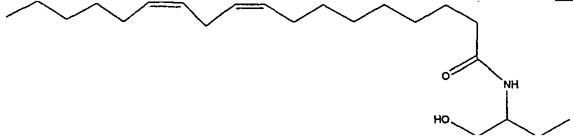 |
| Exemple 3 | 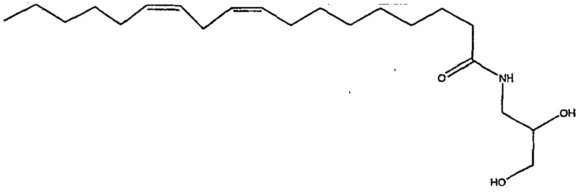 |

### • Exemple 1 : Synthèse du (2-hydroxy-propyl) amide de l'acide octadéca-9,12-cis-cis-diènoïque

Dans un ballon de 500mL, on introduit respectivement 75,11g de 1-amino-2-propanol (1 mole) et 280,24g d'acide linoléique (1 mole). Le mélange sous agitation est chauffé à 65°C avant d'introduire 5g de Novozym® 435. Le milieu réactionnel est ensuite placé sous pression réduite (50 mbars) pour éliminer l'eau formée.

Après 20 heures de synthèse, l'enzyme immobilisée est éliminée par filtration. La conversion de l'acide linoléique en amide est supérieure à 95%. Le produit obtenu se présente sous forme d'une cire de couleur beige.

Le produit obtenu a été analysé par Chromatographie Liquide à Haute Performance (HPLC) selon la méthode habituelle et dans les conditions suivantes :
- Colonne Nucleosil 100 C₁₈ 5 µm (250 x 2 mm)
- Gradient :

| **Temps (minutes)** | **Méthanol (%)** | **Eau (%)** |
|---|---|---|
| 0 | 85 | 15 |
| 10 | 85 | 15 |
| 20 | 100 | 0 |
| 40 | 100 | 0 |
| 45 | 85 | 15 |

- Débit: 0,22 ml.mn⁻¹.
- Détection : Ultra violet λ=210 nm. Temps de rétention du produit en HPLC: 11,98 minutes

### • Exemple 2 : Synthèse du(1-hydroxyméthylpropyl)amide de l'acide octadéca-9,12-cis-diènoïque

Dans un ballon de 500ml, on introduit respectivement 89,11g de 2-amino-1-butanol (1 mole) et 280,24g d'acide linoléique (1 mole). Le mélange sous agitation est chauffé à 65°C avant d'introduire 5g de Novozym® 435. Le milieu réactionnel est ensuite placé sous pression réduite (50 mbars) pour éliminer l'eau formée.
Après 20 heures de synthèse, l'enzyme est éliminée par filtration. La conversion de l'acide linoléique en amide est supérieure à 95%. Le produit obtenu se présente sous forme d'une cire de couleur beige.
Temps de rétention du produit en HPLC : 13,20 minutes

### • Exemple 3: Synthèse du(2,3-dihydroxy-propyl) amide de l'acide octadéca-9,12-cis-cis-diènoïque

Dans un ballon de 500ml, on introduit respectivement 91,11g de 3-amino-1,2-propane diol (1 mole) et 280,24g d'acide linoléique (1 mole). Le mélange sous agitation est chauffé à 65°C avant d'introduire les 5g de Novozym® 435. Le milieu réactionnel est ensuite placé sous pression réduite (50 mbars) pour éliminer l'eau formée.
Après 20 heures de synthèse, l'enzyme est éliminée par filtration. La conversion de l'acide linoléique en amide est supérieure à 95%. Le produit obtenu se présente sous forme d'une cire de couleur beige.
Temps de rétention du produit en HPLC : 10,65 minutes
IR (ν, Cm⁻¹, CH₂Cl₂) : 3300, 2900, 2850, 1630,1545,1465.

### II . Exemples de synthèses d'amides à partir de différents esters d'acide gras en présence de 3-Amino 1,2-propane diol.

| | |
|---|---|
| Exemple 4 | 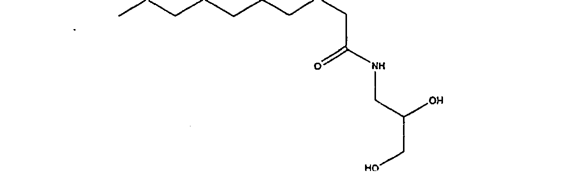 |
| Exemple 5 | 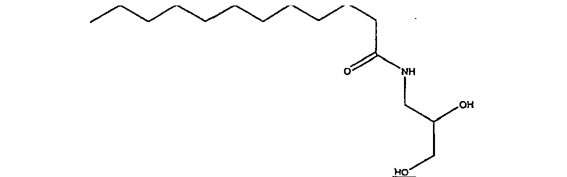 |
| Exemple 6 | 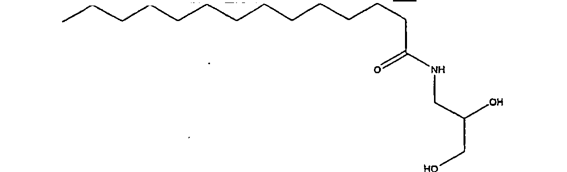 |
| Exemple 7 | 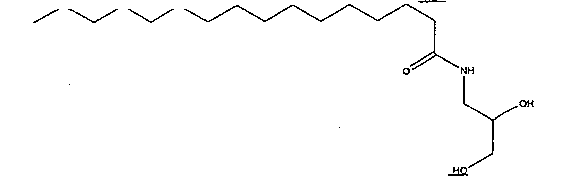 |
| Exemple 8 | 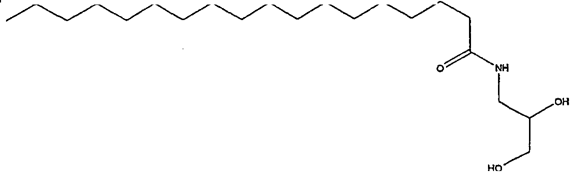 |
| Exemple 9 | 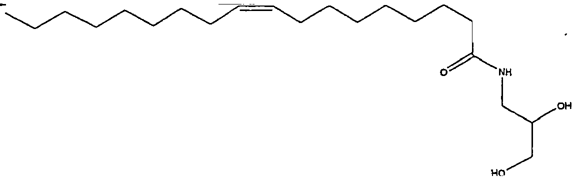 |
| Exemple 10 | 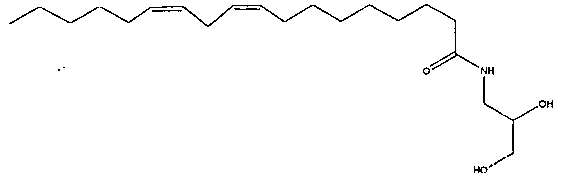 |
| Exemple 11 | 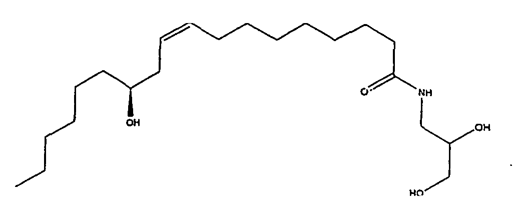 |

### • Exemple 4 : Synthèse du(2,3-dihydroxy-propyl) amide d'acide décanoïque

- Dans un ballon de 500ml, on introduit respectivement 91,11g de 3-amino-1,2-propane diol (1 mole) et 200,32g de décanoate d'éthyle (1 mole). Le mélange sous agitation est chauffé à 65°C avant d'introduire 5g de Novozym® 435: Le milieu réactionnel est ensuite placé sous pression réduite (50 mbars) pour éliminer l'éthanol formé.
- Après 24 heures de synthèse, l'enzyme est éliminée par filtration. La conversion du décanoate d'éthyle en amide est supérieure à 99%. Le produit obtenu se présente sous forme d'une cire de couleur crème.
- Temps de rétention du produit en HPLC : 4,47 minutes

### • Exemple 5 : Synthèse du (2,3-dihydroxy-propyl) amide d'acide dodécanoïque

- Comme dans l'exemple 4, dans un ballon de 500ml, on introduit respectivement 91,11g de 3-amino-1,2-propane diol (1 mole) et 228,37g de laurate d'éthyle (1 mole). Le mélange sous agitation est chauffé à 65°C avant d'introduire les 5g de Novozym® 435. Le milieu réactionnel est ensuite placé sous pression réduite (50 mbars) pour éliminer l'éthanol formé.
- Après 24 heures de synthèse, l'enzyme est éliminée par filtration. La conversion du dodécanoate d'éthyle en amide est supérieure à 99%. Le produit obtenu se présente sous forme d'une cire de couleur crème.
- Temps de rétention du produit en HPLC : 5,65 minutes

### • Exemple 6 : Synthèse du (2,3-dihydroxy-propyl) amide d'acide tétradécanoïque

- Dans un ballon de 500ml, on introduit respectivement 91,11g de 3-amino-1,2-propane diol (1 mole) et 256,42g de myristate d'éthyle (1 mole). Le mélange sous agitation est chauffé à 70°C avant d'introduire 5g de Novozym® 435. Le milieu réactionnel est ensuite placé sous pression réduite (50 mbars) pour éliminer l'éthanol formé.
- Après 24 heures de synthèse, l'enzyme est éliminée par filtration. La conversion du myristate d'éthyle en amide est supérieure à 99%.,Le produit obtenu se présente sous forme d'une cire de couleur crème.
- Temps de rétention du produit en HPLC : 8,06 minutes

### • Exemple 7: Synthèse du(2,3-dihydroxy-propyl) amide d'acide hexadécanoïque

- Dans un ballon de 500ml, on introduit respectivement 91,11g de 3-amino-1,2-propane diol (1 mole) et 284,48g de palmitate d'éthyle (1 mole). Le mélange sous agitation est chauffé à 75°C avant d'introduire 5g de Novozym® 435. Le milieu réactionnel est ensuite placé sous pression réduite (50 mbars) pour éliminer l'éthanol formé.
- Après 24 heures de synthèse, l'enzyme est éliminée par filtration. La conversion du palmitate d'éthyle en amide est supérieure à 99%. Le produit obtenu se présente sous forme d'une cire de couleur crème.
- Temps de rétention du produit en HPLC : 12,15 minutes

### • Exemple 8: Synthèse du(2,3-dihydroxy-propyl) amide d'acide octadécanoïque

- Dans un ballon de 500ml, on introduit respectivement 91,11g de 3-amino-1,2-propane diol (1 mole) et 312,53g de stéarate d'éthyle (1 mole). Le mélange sous agitation est chauffé à 85°C avant d'introduire 5g de Novozym® 435. Le milieu réactionnel est ensuite placé sous pression réduite (50 mbars) pour éliminer l'éthanol formé.
- Après 24 heures de synthèse, l'enzyme est éliminée par filtration. La conversion du stéarate d'éthyle en amide est supérieure à 99%. Le produit obtenu se présente sous forme d'une cire de couleur beige.
- Temps de rétention du produit en HPLC : 19,38 minutes

### • Exemple 9: Synthèse du (2,3-dihydroxy-propyl) amide d'acide octadéc-9-cis-ènoïque

- Dans un ballon de 500ml, on introduit respectivement 91,11g de 3-amino-1,2-propane diol (1 mole) et 310,51g d'oléate d'éthyle (1 mole). Le mélange sous agitation est chauffé à 65°C avant d'introduire les 5g de Novozym® 435. Le milieu réactionnel est ensuite placé sous pression réduite (50 mbars) pour éliminer l'éthanol formé.
- Après 24 heures de synthèse, l'enzyme est éliminée par filtration. La conversion de l'oléate d'éthyle en amide est supérieure à 99%. Le produit obtenu se présente sous forme d'une cire de couleur beige.
- Temps de rétention du produit en HPLC : 13,26 minutes

### • Exemple 10: Synthèse du (2,3-dihydroxy-propyl) amide d'acide octadéca-9,12-cis-cis-diènoïque

- Dans un ballon de 500ml, on introduit respectivement 91,11g de 3-amino-1,2-propane diol (1 mole) et 308,50g de linoléate d'éthyle (1 mole). Le mélange sous agitation est chauffé à 65°C avant d'introduire 5g de Novozym® 435. Le milieu réactionnel est ensuite placé sous pression réduite (50 mbars) pour éliminer l'éthanol formé.
- Après 24 heures de synthèse, l'enzyme est éliminée par filtration. La conversion du linoléate d'éthyle en amide est supérieure à 99%. Le produit obtenu se présente sous forme d'une cire de couleur beige.
- Temps de rétention du produit en HPLC : 10,37 minutes
- IR (ν, Cm⁻¹, CH₂Cl₂) : 3300, 2900, 2850, 1630,1545,1465.

### • Exemple 11: Synthèse du(2,3-dihydroxy-propyl) amide d'acide 12-hydroxy-octadéc-9-ènoïque

- Dans un ballon de 500ml, on introduit respectivement 91,11g de 3-amino-1,2-propane diol (1 mole) et 326,51g de ricinoléate d'éthyle (1 mole). Le mélange sous agitation est chauffé à 65°C avant d'introduire 5g de Novozym® 435. Le milieu réactionnel est ensuite placé sous pression réduite (50 mbars) pour éliminer l'éthanol formé.
- Après 24 heures de synthèse, l'enzyme est éliminée par filtration. La conversion du ricinoléate d'éthyle en amide est supérieure à 99%. Le produit obtenu se présente sous forme d'une cire de couleur beige.
- Temps de rétention du produit en HPLC : 5,34 minutes

### III . Exemples de synthèses des composés de type céramides par réaction d'un ester d'acide gras sur l'amide issu de la condensation de l'acide linoléique sur le 3-Amino 1,2-propane diol.

| | |
|---|---|
| Exemple 12 | 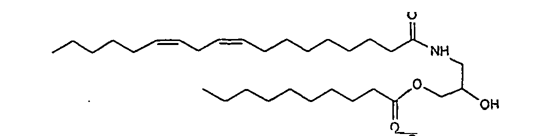 |
| Exemple 13 | 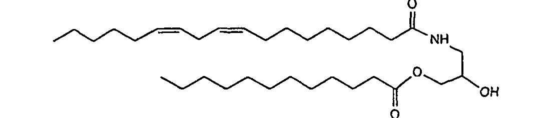 |
| Exemple 14 | 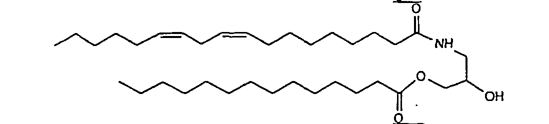 |
| Exemple 15 | 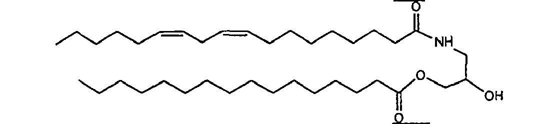 |
| Exemple 16 | 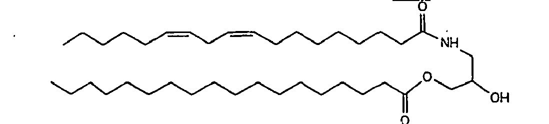 |
| Exemple 17 | 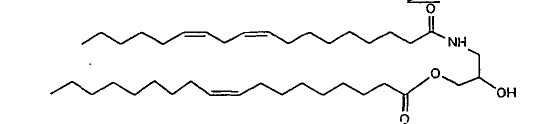 |
| Exemple 18 | 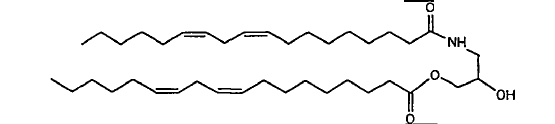 |
| Exemple 19 | 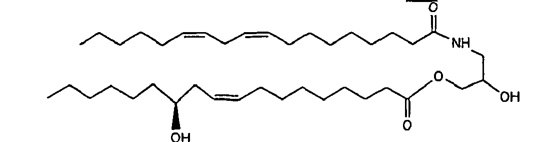 |
| Exemple 20 | 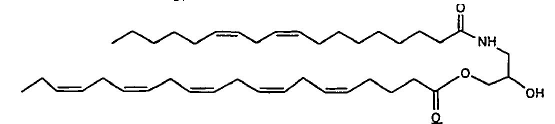 |
| Exemple 21 | 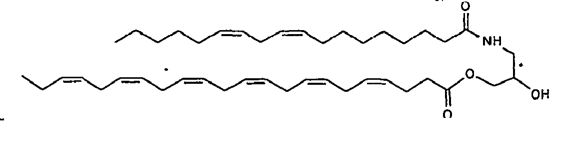 |

### • Exemple 12: Synthèse du (3-decanoyloxy)-2-hydroxy propyl amide de l'acide octadéca-9,12-cis-cis-diènoïque

- Dans un ballon de 1000ml, on introduit respectivement 353,54g de (2,3-dihydroxy-propyl) amide d'acide octadéca-9,12-cis-cis-diènoïque (1 mole) et 300,48g de décanoate d'éthyle (1,5 moles). Le mélange sous agitation est chauffé à 65°C avant d'introduire 30g de Lipozyme® RM IM. Le milieu réactionnel est ensuite placé sous pression réduite (50 mbars) pour éliminer l'éthanol formé.
- Après 24 heures de synthèse, l'enzyme immobilisée est éliminée par filtration. La conversion du 2,3-dihydroxy-propyl amide d'acide linoléique en dérivé N-décanoylé est supérieure à 93%. Le produit obtenu se présente sous forme d'une cire de couleur blanche.
- Temps de rétention du produit en HPLC : 25,35 minutes

### • Exemple 13: Synthèse du (3-dodecanoyloxy)-2-hydroxy propyl amide de l'acide octadéca-9,12-cis-cis-diènoïque

- Dans un ballon de 1000ml, on introduit respectivement 353,54g de (2,3-dihydroxy-propyl) amide d'acide octadéca-9,12-cis-cis-diènoïque (1 mole) et 342,55g de laurate d'éthyle (1,5 moles). Le mélange sous agitation est chauffé à 65°C avant d'introduire les 30g de Lipozyme® RM IM. Le milieu réactionnel est ensuite placé sous pression réduite (50 mbars) pour éliminer l'éthanol formé.
- Après 24 heures de synthèse, l'enzyme est éliminée par filtration. La conversion du (2,3-dihydroxy-propyl) amide d'acide linoléique est supérieure à 93%. Le produit obtenu se présente sous forme d'une cire de couleur blanche.
- Temps de rétention du produit en HPLC :27,28 minutes

### • Exemple 14: Synthèse du (3-tétradécanoyloxy)-2-hydroxy propyl amide de l'acide octadéca-9,12-cis-cis-diènoïque

- Dans un ballon de 1000ml, on introduit respectivement 353,54g de (2,3-dihydroxy-propyl) amide d'acide octadéca-9,12-cis-cis-diènoïque (1 mole) et 384,63g de myristate d'éthyle (1,5 moles). Le mélange sous agitation est chauffé à 65°C avant d'introduire 30g de Lipozyme® RM IM. Le milieu réactionnel est ensuite placé sous pression réduite (50 mbars) pour éliminer l'éthanol formé.
- Après 24 heures de synthèse, l'enzyme est éliminée par filtration. La conversion du (2,3-dihydroxy-propyl) amide d'acide linoléique est supérieure à 93%. Le produit obtenu se présente sous forme d'une cire de couleur blanche.
- Temps de rétention du produit en HPLC : 28,93 minutes

### • Exemple 15: Synthèse du (3-hexadécanoyloxy)-2-hydroxy propyl amide de l'acide octadéca-9,12-cis-cis-diènoïque

- Dans un ballon de 1000ml, on introduit respectivement 353,54g de (2,3-dihydroxy-propyl) amide d'acide octadéca-9,12-cis-cis-diènoïque (1 mole) et 426,72g de palmitate d'éthyle (1,5 moles). Le mélange sous agitation est chauffé à 65°C avant d'introduire les 30g de Lipozyme® RM IM. Le milieu réactionnel est ensuite placé sous pression réduite (50 mbars) pour éliminer l'éthanol formé.
- Après 24 heures de synthèse, l'enzyme est éliminée par filtration. La conversion du (2,3-dihydroxy-propyl) amide d'acide linoléique est supérieure à 93%. Le produit obtenu se présente sous forme d'une cire de couleur blanche.
- Temps de rétention du produit en HPLC : 31,22 minutes
- IR (ν, Cm⁻¹, CH₂Cl₂) : 3300, 2900, 2850, 1695, 1630, 1540, 1460.

### • Exemple 16: Synthèse du(3-octadécanoyloxy)-2-hydroxy propyl amide de l'acide octadéca-9,12-cis-cis-diènoïque

- Dans un ballon de 1000ml, on introduit respectivement 353,54g de (2,3-dihydroxy-propyl) amide d'acide octadéca-9,12-cis-cis-diènoïque (1 mole) et 468,79g de stéarate d'éthyle (1,5 moles). Le mélange sous agitation est chauffé à 65°C avant d'introduire les 30g de Lipozyme® RM IM. Le milieu réactionnel est ensuite placé sous pression réduite (50 mbars) pour éliminer l'éthanol formé.
- Après 24 heures de synthèse, l'enzyme est éliminée par filtration. La conversion du (2,3-dihydroxy-propyl) amide d'acide linoléique est supérieure à 93%. Le produit obtenu se présente sous forme d'une cire de couleur blanche.
- Temps de rétention du produit en HPLC : 33,57 minutes

### • Exemple 17: Synthèse du (3-octadéca-9-iénoyloxy)-2-hydroxy propyl amide de l'acide octadéca-9,12-cis-cis-diènoïque

- Dans un ballon de 1000ml, on introduit respectivement 353,54g de (2,3-dihydroxy-propyl) amide d'acide octadéca-9,12-cis-cis-diènoïque (1 mole) et 465,76g d'oléate d'éthyle (1,5 moles). Le mélange sous agitation est chauffé à 65°C avant d'introduire les 30g de Lipozyme® RM IM. Le milieu réactionnel est ensuite placé sous pression réduite (50 mbars) pour éliminer l'éthanol formé.
- Après 24 heures de synthèse, l'enzyme est éliminée par filtration. La conversion du (2,3-dihydroxy-propyl) amide d'acide linoléique est supérieure à 93%. Le produit obtenu se présente sous forme d'une cire de couleur crème.
- Temps de rétention du produit en HPLC : 31,73 minutes

### • Exemple 18: Synthèse du (3-octadéca-9,12-cis-cis-diènoyloxy)-2-hydroxy propyl amide de l'acide octadéca-9,12-cis-cis-diènoïque

- Dans un ballon de 1000ml, on introduit respectivement 353,54g de (2,3-dihydroxy-propyl) amide d'acide octadéca-9,12-cis-cis-diènoïque (1 mole) et 462,75g de linoléate d'éthyle (1,5 moles). Le mélange sous agitation est chauffé à 65°C avant d'introduire les 30g de Lipozyme® RM IM. Le milieu réactionnel est ensuite placé sous pression réduite (50 mbars) pour éliminer l'éthanol formé.
- Après 24 heures de synthèse, l'enzyme est éliminée par filtration. La conversion du (2,3-dihydroxy-propyl) amide d'acide linoléique est supérieure à 93%. Le produit obtenu se présente sous forme d'une cire de couleur crème.
- Temps de rétention du produit en HPLC : 30,19 minutes

### • Exemple 19: Synthèse du(3-octadéca-12-hydroxy-9 diènoyloxy)-2-hydroxy propyl amide de l'acide octadéca-9,12-cis-cis-diènoïgue

- Dans un ballon de 1000ml, on introduit respectivement 353,54g de (2,3-dihydroxy-propyl) amide d'acide octadéca-9,12-cis-cis-diènoïque (1 mole) et 489,76g de ricinoléate d'éthyle (1,5 moles). Le mélange sous agitation est chauffé à 65°C avant d'introduire les 30g de Lipozyme® RM IM. Le milieu réactionnel est ensuite placé sous pression réduite (50 mbars) pour éliminer l'éthanol formé.
- Après 24 heures de synthèse, l'enzyme est éliminée par filtration. La conversion du (2,3-dihydroxy-propyl) amide d'acide linoléique est supérieure à 93%. Le produit obtenu se présente sous forme d'une cire de couleur de couleur crème.
- Temps de rétention du produit en HPLC : 26,05 minutes

### • Exemple 20: Synthèse du (3-éicosa-5,8,11,14,17-pentaènoyloxy)-2-hydroxy propyl amide de l'acide octadéca-9,12-cis-cis-diènoïque

- Dans un ballon de 1000ml, on introduit respectivement 353,54g de (2,3-dihydroxy-propyl) amide d'acide octadéca-9,12-cis-cis-diènoïque (1 mole) et 495,75g d'Icosa-5,8,11,14,17-pentaènoïque éthyl ester (1,5 moles). Le mélange sous agitation est chauffé à 65°C avant d'introduire les 30g de Lipozyme® RM IM. Le milieu réactionnel est ensuite placé sous pression réduite (50 mbars) pour éliminer l'éthanol formé.
- Après 24 heures de synthèse, l'enzyme est éliminée par filtration. La conversion du (2,3-dihydroxy-propyl) amide d'acide linoléique est supérieure à 93%. Le produit obtenu se présente sous forme d'une cire de couleur crème.
- Temps de rétention du produit en HPLC : 28,60 minutes

### • Exemple 21 : Synthèse du (3-docosa-4,7,10,16,19-hexaènoyloxy)-2-hydroxy propyl amide de l'acide octadéca-9,12-cis-cis-diènoïque

- Dans un ballon de 1000ml, on introduit respectivement 353,54g de (2,3-dihydroxy-propyl) amide d'acide octadéca-9,12-cis-cis-diènoïque (1 mole) et 534,9g de docosa-5,8,11,14,17-hexaènoïque éthyl ester (1,5 moles). Le mélange sous agitation est chauffé à 65°C avant d'introduire les 30g de Lipozyme® RM IM. Le milieu réactionnel est ensuite placé sous pression réduite (50 mbars) pour éliminer l'éthanol formé.
- Après 24 heures de synthèse, l'enzyme est éliminée par filtration. La conversion du (2,3-dihydroxy-propyl) amide d'acide linoléique est supérieure à 93%. Le produit obtenu se présente sous forme d'une cire de couleur crème.
- Temps de rétention du produit en HPLC : 29,37 minutes

### IV . Exemples de compositions cosmétiques incorporant les composés de formules I :

Ces préparations ont été obtenues par mélange des ingrédients.

Dans les exemples et les préparations, les proportions indiquées sont des pourcentages en poids/poids total

### • Emulsion-Crème huile dans eau

| | |
|---|---|
| Huile minérale | 4,00% |
| Composé de type céramide de formule (I) | 0.10% |
| Ceteth 10 | 4,00% |
| Cetyl alcohol | 4,00% |
| Triethanolamine | 0.75% |
| Butane-1.3-diol | 3.00% |
| Gomme xanthane | 0.30% |
| conservateur | 0.40% |
| Parfum | qs |
| Hydroxy toluène butylè | 0.01 % |
| Eau | qsp 100% |

### • Lotion pour cheveux secs

| | |
|---|---|
| Composé de type céramide selon la formule (I) | 1.5% |
| Parfum | 0.10% |
| Hydroxyethyle cellulose | 0.40% |
| Ethanol absolu | 25.00% |
| Benzoate de p-methyle sodé | 0.20% |
| Eau déminéralisée stérile | qsp 100% |

### • Lotion hydratante et anti-âge pour peaux sèches

| | |
|---|---|
| Composé de type céramide selon la formule (I) | 1.5% |
| Parfum | 0.10% |
| Hydroxyethyl cellulose | 0.40% |
| Ethanol absolu | 25.00% |
| Benzoate de p-methyle. | 0.20% |
| Eau déminéralisée stérile | qsp 100% |

### • Lotion hydratante et anti-âge pour peaux sèches

| | |
|---|---|
| Composé de type céramide selon la formule (I) | 0.25% |
| Ethanol | 10.00% |
| Parfum | 0.50% |
| Conservateur | 0.40 |
| Eau déminéralisée stérile | qsp 100% |

### • Lotion alcoolique pour le soin des ongles

| | |
|---|---|
| Composé de type céramide selon la formule (I) | 0.20% |
| Diméthylsulfoxyde | 10.00% |
| Ethanol | 40.00% |
| Anti-oxydant | 0.10% |
| Parfum | qs |
| Eau déminéralisée stérile | qsp 100% |

## Revendications

1. Procédé de synthèse de composés de type céramide comportant au moins une étape d'amidification effectuée par l'enzyme de type lipase B de *Candida antartica* et une étape d'estérification, également réalisée par une enzyme de type lipase, lesdits composés de type céramide étant de formule générale (I) : 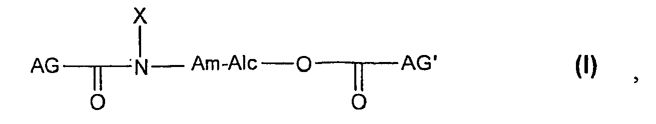 dans laquelle le groupement Am-Alc représente une chaîne hydrocarbonée, préférentiellement saturée, linéaire, éventuellement ramifiée, comportant de 2 à 6 atomes de carbone, issue d'un amino-alcool ; X représente un hydrogène ou une chaîne carbonée contenant de 1 à 4 atomes de carbone, éventuellement hydroxylée sur les carbones en position 2' et/ou suivants de la fonction amine; et les groupements notés AG et AG' désignent chacun une chaîne carbonée, saturée ou insaturée, comportant de 4 à 30 atomes de carbone, issue d'un acide gras et/ou d'un ester d'acides gras ; les deux groupements AG et AG' pouvant être identiques ou différents.

2. Procédé de synthèse selon la revendication 1, **caractérisé en ce que** la réaction d'amidification est réalisée en conditions stoechiométriques entre un acide gras et/ou son ester, et un amino-alcool et à une température comprise entre 40 et 100°C.

3. Procédé de synthèse selon l'une des revendications 1 ou 2, **caractérisé en ce que** l'amidification est effectuée en l'absence de solvant à une température minimale d'environ 65°C.

4. Procédé de synthèse selon l'une des revendications 1 à 3, **caractérisé en ce que** l'amidification est effectuée, sous un vide partiel compris entre 500 et 1 mbars, et pendant au moins 16 heures

5. Procédé de synthèse selon l'une des revendications 1 à 4, **caractérisé en ce que** l'estérification est effectuée par la lipase de *Rhizomucor miehei.*

6. Procédé de synthèse selon la revendication 5, **caractérisé en ce que** la réaction d'estérification est effectuée avec un ratio ester d'acide gras sur amino-alcool compris entre 1 et 2.

7. Procédé de synthèse selon l'une des revendications 5 ou 6, **caractérisé en ce que** la réaction d'estérification est effectuée et à une température comprise entre 40 et 90°C.

8. Procédé de synthèse selon l'une des revendications 5 à 7, **caractérisé en ce que** la réaction d'estérification est effectuée sans solvant, à une température minimale d'environ 65°C.

9. Procédé de synthèse selon l'une des revendications 5 à 8, **caractérisé en ce que** la réaction d'estérification est effectuée sous un vide partiel compris entre 500 et 1 mbars et pendant au moins 18 heures.

10. Procédé de synthèse selon l'une des revendications 1 à 9, **caractérisé en ce que** les enzymes utilisées pour chaque étape sont immobilisées sur un support inerte.

11. Procédé de synthèse selon l'une des revendications 1 à 11, **caractérisé en ce que** les réactions d'amidification par la lipase B de *Candida antartica* et d'estérification par la lipase de *Rhizomucor miehei* sont effectuées toutes deux sans solvant, éventuellement simultanément, à une température minimale d'environ 65°C et sous un vide partiel compris entre 30 et 200 mbars.

12. Procédé de synthèse selon l'une des revendications 1 à 11, **caractérisé en ce que** les amino-alcools sont des composés, de préférence saturés, linéaires, éventuellement ramifiés, comportant de 2 à 6 atomes de carbone et les acides gras et/ou esters d'acides gras ont une chaîne carbonée, saturée ou insaturée, éventuellement hydroxylée, et contenant de 4 à 30 atomes de carbone, de préférence de 10 à 22 atomes.

13. Procédé de synthèse selon l'une des revendications 1 à 12, **caractérisé en ce que** l'amino-alcool initial possède la formule (IV): 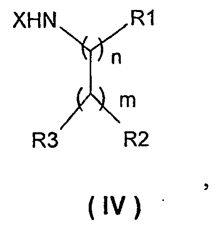 dans laquelle :
- n est un nombre entier choisi parmi les valeurs 1, 2, 3 et m est un nombre entier choisi parmi les valeurs 1, 2 et 3.
- X est choisi parmi l'hydrogène et une chaîne carbonée contenant de 1 à 4 atomes de carbone, éventuellement hydroxylée sur les carbones en position 2' et/ou suivants de la fonction amine,
- R1 est choisi parmi l'hydrogène et une chaîne alkyle comportant de 1 à 4 atomes de carbone, préférentiellement saturée, linéaire, éventuellement ramifiée et/ou hydroxylée,
- R2 est choisi parmi l'hydrogène, un hydroxyle, un groupement NH₂ et une chaîne alkyle comportant de 1 à 4 atomes de carbone, préférentiellement saturée, linéaire, éventuellement ramifiée et/ou hydroxylée,
- R3 est choisi parmi l'hydrogène, un hydroxyle et un groupement CH₂OH,
et dans laquelle, au moins un des groupements R1, R2 ou R3 contient un groupement hydroxyle.

14. Procédé de synthèse selon l'une des revendications 1 à 13, **caractérisé en ce que** l'étape d'amidification est effectuée avant l'étape d'estérification.

## Claims

1. A process for the synthesis of ceramide like compounds including at least one step of amidification achieved by the enzyme of lipase B type from Candida antartica and one step of esterification also achieved by an enzyme of lipasetype, said ceramide like compounds having the general formula (I) 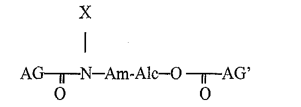 wherein the grouping Am-Alc represents a straight, optionally branched hydrocarbonchain, having from 2 to 6 carbon atoms, stemming from an amino alkanol X represents a hydrogen or a carbon chain including from 1 to 4 carbon atoms, optionally hydroxylated on the carbons in position 2 and/or further of the amine function and the grouping defined as AG and AG' each designated a carbon chain, saturated or unsaturated, having from 4 to 30 carbon atoms, stemming from a fatty acid and/or an ester of fatty acid, the two groupings being the same or different.

2. A process for the synthesis according to claim 1, wherein the amidification reaction is achieved under stoechiometric conditions between a fatty acid and/or an ester thereof and an amino alkanol at a temperature ranging between 40 and 100°C.

3. A process for the synthesis according to any of claim 1 or 2, wherein amidification is performed in the absence of solvent, at a temperature of at least about 65°C.

4. A process for the synthesis according to any of claim 1 or 2, wherein amidification is performed under a partial vacuum ranging from 500 to 1 m bar during at least 16 hours.

5. A process for synthesis according to anyone of claims 1 to 4, wherein the esterification is performed using the lipase from Rhizomucor miehei.

6. A process for synthesis according to claim 5, wherein the esterification reaction is performed with a ratio fatty acid ester to amino alkanol ranging from 1 to 2.

7. A process for synthesis according to one of claims 5 or 6 wherein the esterification reaction is performed at a temperature comprised between 40 and 90° C.

8. A process for synthesis according to tone of claims 5 to 7 wherein the esterification reaction is performed without a solvent, at a temperature of about 65°C.

9. A process of synthesis according to one of the claims 5 to 8, wherein the esterification reaction is performed under partial vacuum comprised between 500 et 1 m bar during at least 18 hours.

10. A process for synthesis according to anyone of claims 1 to 9, wherein the enzymes utilized for each step are immobilized on an inert support.

11. A process for synthesis according to anyone of claims 1 to 11, wherein the amidification reaction is performed by means of the lipase B from Candida antartica, and the esterification reaction is performed by means of the lipase from Rhizomucor, optionally simultaneously, at a temperature of at least about 65°C and under a partial vacuum comprised between 30 and 200 m bars.

12. A process for synthesis according to anyone of claims 1 to 11, wherein the amino alkanols are preferably saturated, with straight chain optionally branched, compounds having from 2 to 6 carbon atoms and the fatty acids and/or esters of fatty acids have a saturated or unsaturated, optionally hydroxylated carbon chain including from 4 to 30 carbon atoms, preferably from 10 to 22 carbon atoms.

13. A process for synthesis according to anyone of claims 1 to 12, wherein the aminoalkanol has the formula IV 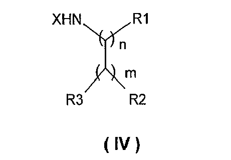 wherein
n is an integer selected among the values 1, 2 and 3 and m is an integer selected among the values 1, 2 and 3.
X is selected from the group consisting of hydrogen and a carbon chain having from 1 to 4 carbon atoms, optionally hydroxylated on the carbons in position 2' and/or following from the amine function.
R1 is selected among hydrogen and an alkyl chain having from 1 to 4 carbon atoms, preferably saturated, linear or optionally branched and/or hydroxylated.
R3 is selected from the group consisting of hydrogen, a hydroxyl and a grouping CH2 OH and wherein at least one of the groupings R1, R2 or R3 includes a grouping hydroxyl.

14. A process for synthesis according to anyone of claims 1 to 13, wherein the amidification step is performed before the esterification step.

## Patentansprüche

1. Verfahren zur Synthese von Verbindungen des Ceramid-Typs, umfassend mindestens einen Amidierungsschritt, der durch das Enzym vom Typ Lipase B aus *Candida antartica* ausgeführt wird, und einen Esterifizierungsschritt, der ebenfalls durch ein Enzym vom Typ Lipase durchgeführt wird, wobei die Verbindungen vom Ceramid-Typ die allgemeine Formel (I): 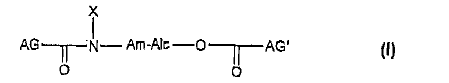 aufweisen, in der die Gruppe Am-Alc eine vorzugsweise gesättigte, lineare, eventuell verzweigte Kohlenwasserstoffkette mit 2 bis 6 Kohlenstoffatomen darstellt, die aus einem Aminoalkohol hervorgegangen ist; X einen Wasserstoff oder eine Kohlenstoffkette mit 1 bis 4 Kohlenstoffen darstellt, die eventuell an den Kohlenstoffatomen an der Position 2' und/oder folgenden der Aminfunktion hydroxyliert ist; und die mit AG und AG' bezeichneten Gruppen jeweils eine gesättigte oder ungesättigte Kohlenstoffkette mit 4 bis 30 Kohlenstoffatomen darstellt, die aus einer Fettsäure und/oder einem Fettsäureester hervorgegangen ist; wobei die beiden Gruppen AG und AG' identisch oder unterschiedlich sein können.

2. Syntheseverfahren nach Anspruch 1, **dadurch gekennzeichnet, dass** die Amidierungsreaktion unter stöchiometrischen Bedingungen zwischen einer Fettsäure und/oder ihrem Ester sowie einem Aminoalkohol und bei einer Temperatur zwischen 40 und 100°C durchgeführt wird.

3. Syntheseverfahren nach einem der Ansprüche 1 oder 2, **dadurch gekennzeichnet, dass** die Amidierung in Abwesenheit eines Lösemittels bei einer Mindesttemperatur von etwa 65°C durchgeführt wird.

4. Syntheseverfahren nach einem der Ansprüche 1 bis 3, **dadurch gekennzeichnet, dass** die Amidierung in einem Teilvakuum zwischen 500 und 1 mbar sowie während mindestens 16 Stunden durchgeführt wird.

5. Syntheseverfahren nach einem der Ansprüche 1 bis 4, **dadurch gekennzeichnet, dass** die Esterifizierung durch die Lipase aus *Rhizomucor miehei* durchgeführt wird.

6. Syntheseverfahren nach Anspruch 5, **dadurch gekennzeichnet, dass** die Esterifizierungsreaktion mit einem Verhältnis Fettsäureester/Aminoalkohol zwischen 1 und 2 durchgeführt wird.

7. Syntheseverfahren nach einem der Ansprüche 5 oder 6, **dadurch gekennzeichnet, dass** die Esterifizierungsreaktion bei einer Temperatur zwischen 40 und 90°C durchgeführt wird.

8. Syntheseverfahren nach einem der Ansprüche 5 bis 7, **dadurch gekennzeichnet, dass** die Esterifizierungsreaktion ohne Lösemittel bei einer Mindesttemperatur von etwa 65°C durchgeführt wird.

9. Syntheseverfahren nach einem der Ansprüche 5 bis 8, **dadurch gekennzeichnet, dass** die Esterifizierungsreaktion in einem Teilvakuum zwischen 500 und 1 mbar sowie während mindestens 18 Stunden durchgeführt wird.

10. Syntheseverfahren nach einem der Ansprüche 1 bis 9, **dadurch gekennzeichnet, dass** die für jeden Schritt verwendeten Enzyme auf einem inerten Träger immobilisiert sind.

11. Syntheseverfahren nach einem der Ansprüche 1 bis 10, **dadurch gekennzeichnet, dass** die Reaktionen der Amidierung durch Lipase B aus *Candida antartica* und der Esterifizierung durch Lipase aus *Rhizomucor miehei* jeweils ohne Lösemittel, eventuell gleichzeitig, bei einer Mindesttemperatur von etwa 65°C und in einem Teilvakuum zwischen 30 und 200 mbar durchgeführt werden.

12. Syntheseverfahren nach einem der Ansprüche 1 bis 11, **dadurch gekennzeichnet, dass** die Aminoalkohole vorzugsweise gesättigte, lineare, eventuell verzweigte Verbindungen mit 2 bis 6 Kohlenstoffatomen sind und die Fettsäuren und/oder Fettsäureester eine gesättigte oder ungesättigte, eventuell hydroxylierte Kohlenstoffkette mit 4 bis 30 Kohlenstoffatomen, vorzugsweise von 10 bis 22 Atomen, aufweisen.

13. Syntheseverfahren nach einem der Ansprüche 1 bis 12, **dadurch gekennzeichnet, dass** der ursprüngliche Aminoalkohol die Formel (IV): 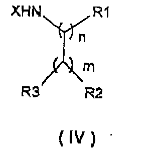 aufweist, in der:
- n eine ganze Zahl ist, die unter den Werten 1, 2, 3 gewählt wird, und m eine ganze Zahl ist, die unter den Werten 1, 2 und 3 gewählt wird,
- X aus Wasserstoff und einer Kohlenstoffkette mit 1 bis 4 Kohlenstoffatomen gewählt wird, die eventuell an den Kohlenstoffen an der Position 2' und/oder folgenden der Aminfunktion hydroxyliert ist,
- R1 aus Wasserstoff und einer vorzugsweise gesättigten, linearen, eventuell verzweigten und/oder hydroxylierten Alkylkette mit 1 bis 4 Kohlenstoffatomen gewählt wird,
- R2 aus Wasserstoff, einem Hydroxyl, einer NH₂-Gruppe und einer vorzugsweise gesättigten, linearen, eventuell verzweigten und/oder hydroxylierten Alkylkette mit 1 bis 4 Kohlenstoffatomen gewählt wird,
- R3 aus Wasserstoff, einem Hydroxyl und einer CH₂OH-Gruppe gewählt wird
und in der mindestens eine der Gruppen R1, R2 oder R3 eine Hydroxylgruppe enthält.

14. Syntheseverfahren nach einem der Ansprüche 1 bis 13, **dadurch gekennzeichnet, dass** der Amidierungsschritt vor dem Esterifizierungsschritt durchgeführt wird.
